(19) Europäisches Patentamt
European Patent Office
Office européen des brevets

(11) **EP 4 539 739 B1**

(12) **EUROPEAN PATENT SPECIFICATION**

(45) Date of publication and mention
of the grant of the patent:
**03.06.2026 Bulletin 2026/23**

(51) International Patent Classification (IPC):
*A61B 5/103* (2006.01)   *A61B 5/11* (2006.01)
*A61B 5/00* (2006.01)   *A61B 7/00* (2006.01)

(21) Application number: **23732926.3**

(22) Date of filing: **15.06.2023**

(52) Cooperative Patent Classification (CPC):
**A61B 5/1036; A61B 5/1111; A61B 5/4557;
A61B 5/6817; A61B 7/006;** A61B 2562/0247

(86) International application number:
**PCT/EP2023/066029**

(87) International publication number:
**WO 2023/242306 (21.12.2023 Gazette 2023/51)**

(54) **A SYSTEM FOR DETECTING BRUXISM OR TEMPOROMANDIBULAR JOINT DISORDERS**

SYSTEM ZUR ERKENNUNG VON BRUXISMUS ODER KIEFERGELENKSTÖRUNGEN

SYSTÈME DE DÉTECTION DU BRUXISME OU DES TROUBLES DE L'ARTICULATION TEMPORO-MANDIBULAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.06.2022 EP 22179360**

(43) Date of publication of application:
**23.04.2025 Bulletin 2025/17**

(73) Proprietor: **Koninklijke Philips N.V.
5656 AE Eindhoven (NL)**

(72) Inventors:
• **HIWALE, Sujitkumar**
**5656 AG Eindhoven (NL)**
• **JOHNSON, Mark Thomas**
**5656 AG Eindhoven (NL)**
• **GERHARDT, Lutz Christian**
**5656AG Eindhoven (NL)**

(74) Representative: **Philips Intellectual Property & Standards
High Tech Campus 52
5656 AG Eindhoven (NL)**

(56) References cited:
**EP-A1- 3 936 046       JP-B2- 3 810 026
US-A1- 2015 039 087    US-A1- 2019 343 452
US-A1- 2020 312 321**

• **ANDO TOSHIYUKI ANDO@IPLAB CS TSUKUBA AC JP ET AL: "CanalSense Face-Related Movement Recognition System based on Sensing Air Pressure in Ear Canals", PROCEEDINGS OF THE 2017 ACM ON CONFERENCE ON INFORMATION AND KNOWLEDGE MANAGEMENT, ACMPUB27, NEW YORK, NY, USA, 20 October 2017 (2017-10-20), pages 679 - 689, XP058541920, ISBN: 978-1-4503-5586-5, DOI: 10.1145/ 3126594.3126649**

**Description**

FIELD OF THE INVENTION

The invention relates to a system for detecting and differentiating between bruxism or temporomandibular joint disorders, in particular in a non-obtrusive and user friendly manner.

BACKGROUND OF THE INVENTION

The jawbones (the upper, maxilla and the lower, mandible) are integral parts of the oral cavity. These bones are important for normal functioning of the oral cavity, and have been also associated with pathological conditions in oral cavity. Bruxism is a condition in which a subject grinds, gnashes or clenches the teeth. Temporomandibular joint disorder is another condition linked with the jawbones.

Bruxism can be categorized as sleep bruxism and awake bruxism. Sleep bruxism is characterized by rhythmic or non-rhythmic muscle activities of the biting apparatus during sleep. Awake bruxism is characterized by repetitive or sustained tooth contact and/or by bracing or thrusting/sliding of the lower jawbone. The prevalence of bruxism varies according to the age group. In the adult population, the prevalence of awake bruxism is estimated to be around 22%-30%, and sleep bruxism around 1%-15% of the population. In children and adolescents the prevalence of bruxism is reported to be around 3%-49%.

Bruxism is associated with a plethora of short and long-term adverse health effects. Short-term effects of bruxism include headache, limitation of mouth opening, facial myalgia (aching jaw & facial muscles), earache, and tightness or stiffness of the shoulders. Bruxism also impacts sleep in the form of frequent disruptions. It is also known that the grinding or tapping noise of the teeth due to bruxism can lead to sleep disruption of a bed partner. The oral manifestations of bruxism include excess tooth mobility and inflamed and receding gums. The long-term effects of bruxism include Temporomandibular Joint Disorders (hereinafter referred to as TJDs, discussed further below), tooth wear and fractures.

If diagnosed and treated early, many bruxism-related problems can be avoided. It has for example been observed that awake bruxism can be reduced by using proper reminder techniques.

The temporomandibular joints (TMJs) are the two joints connecting the mandible (jawbone) to the skull. It is estimated that ~20-40% of population has some degree of TJD. Three classical features of TJD include pain, reduced range of jaw movements and noise (clicking/popping) from the joint.

The ear is divided into three parts; external ear, middle ear, and inner ear. The external ear canal is approximately 25 mm in length with a diameter of around 7 mm. The TMJs are located in the cartilaginous floor of the external ear canal. Thus, movement of the jaw changes the dimension of the floor external ear canal.

The pressure in the middle ear is maintained very close to atmospheric pressure by various physiological mechanisms including airflow through the auditory tube. Normally, the auditory tube is collapsed, but it gapes open both with swallowing and with other oral activities, which creates a positive pressure in the nasal cavity. Hence, movements in the oral cavity are likely to produce characteristic changes in external ear pressure.

It would be desirable to be able to diagnose bruxism or TJD early, for example to enable a timely alert to be provided to a user to stop teeth grinding or clenching.

US20150039087A1 describes an in-ear device for managing temporomandibular joint-related symptoms.

US2019343452A1 describes a bruxism detection subsystem positioned at least partly in the ear canal capable of measuring a change in a size of the ear canal of a patient.

EP3936046A1 describes a system for assessing ear pathologies by analyzing passive pressure changes in the ear canal, induced by subject maneuvers.

JP3810026B2 describes a system and method for measuring middle ear dynamics associated with mandibular movement by detecting ear canal pressure and jaw position to quantitatively assess mandibular function.

US2020312321A1 describes a system for classifying nonverbal audio events, such as teeth grinding or swallowing, by capturing and analyzing in-ear audio signals with an electronic earpiece.

SUMMARY OF THE INVENTION

The invention is defined by the claims.

According to examples in accordance with an aspect of the invention, there is provided a system for detecting and distinguishing between bruxism and temporomandibular joint disorders, comprising:

>     a pressure sensor arrangement for sensing an external ear canal pressure; and
>     a processor for processing the sensed ear canal pressure, wherein the processor is configured to:
>
>>         detect bruxism or a temporomandibular joint disorder from the sensed ear canal pressure; and
>>         generate an output signal indicating the detected bruxism or temporomandibular joint disorders.

This system monitors pressure changes in the external ear canal to detect oral pathologies such as bruxism and TJD. The output signal is for example used to deliver appropriate notifications or reminders to a user based on the detected pathology.

The invention is based on the recognition that it is possible to use characteristics of a measured external ear pressure to detect and quantify typical movements involving the jawbones such as jaw up, jaw down, and side-to-side movement of the jaw. This information can then be further used to detect complex movement of oral cavity such as swallowing and yawning, which includes different combinations of typical movement of jawbones.

Based on the properties of these movements, it can be detected if there is bruxism or temporomandibular joint disorders and the system can distinguish between them.

The pressure sensor may comprise a speaker which is operated in a microphone pressure sensing mode. The speaker is for example an existing part of an earbud, for example of an entertainment system or a sleep monitoring system. It may be part of a hearing aid system. A single speaker may be used in time division mode as a speaker and as a pressure sensor. There may instead be two loudspeaker transducers; one is used as pressure sensor and the other is used as a speaker. Alternatively pressure sensors may be used which do not have output (e.g. loudspeaker) function.

The system may comprise a microphone for audio detection and the processor is for performing audio analysis. The processor may for example be further configured to detect clicking or popping sounds or sounds emerging from teeth grinding or clicking sounds relating to temporomandibular joint disorders. Thus, the analysis of sounds made as well as pressure levels may assist in detecting bruxism or temporomandibular joint disorders.

The pressure sensor arrangement is configured for obtaining a differential pressure measurement from the two ears, either simultaneously (e.g. with two pressure sensing earbuds) or sequentially.

For example, constant differences may relate to ear/nose/throat/sinus pathologies rather than a bruxism signal (which is periodic in nature). Furthermore, differentiation in arrival time (phase) or amplitude of the signals may enable pathologies to be identified more reliably. Thus, the processor is configured to use the differential pressure measurement to differentiate between (i) bruxism and temporomandibular joint disorders and (ii) and car/nose/sinus pathologies.

The processor may be configured to analyze the sensed ear canal pressure to detect characteristic pressure patterns and repetition rates. Thus, both pressure waveforms over a short time period (corresponding to one grinding event, for example) and repetition rates may be used.

The processor may be configured to detect bruxism by:

> identifying types of jaw movement from the sensed ear canal pressure;
> determine if grinding or clenching jaw movements are present; and

detect bruxism based on a frequency of grinding or jaw clenching movements which exceeds a threshold.

Thus, jaw grinding or jaw clenching movements are in this way detected and counted.

The processor may be configured to detect temporomandibular joint disorders by:

> identifying types of jaw movement from the sensed ear canal pressure;
> determine if jaw closing movements are present;
> determine a jaw movement range and jaw movement speed from pressure change amplitudes and rate of change of pressure changes; and
> detect temporomandibular joint disorders based on a function of a jaw movement range and jaw movement speed.

Thus, jaw movement ranges and speeds are analyzed.

The processor may be configured to analyze the sensed ear canal pressure to detect whether the user is awake or asleep. This for example enables sleep bruxism and awake bruxism to be differentiated.

The system may further comprise a reminder system for providing reminders or notifications to the user indicating the detected bruxism or temporomandibular joint disorders. This is known way to treat the condition.

The invention also provides an oral care system comprising:

> an oral care head; and
> the system as defined above.

In this oral care system, an oral healthcare routine such as tooth brushing, jetting or flossing, may be used, in particular for the measurement of TJD. For example, there will be opening of the mouth at the start of a tooth brushing session and closing of the mouth at the end of a tooth brushing session. Similarly, there will be opening of the mouth to insert a brushing mouthpiece and also to remove a brushing mouthpiece, and closing the mouth down onto a brushing mouthpiece after it has been inserted. Opening of the mouth will also be needed to access the rear teeth for jetting or flossing, as well as possible side-to-side motion when jetting or flossing the rear teeth.

The disclosure also provides a computer program comprising computer program code means which is adapted, when said program is run on a computer, to implement a method comprising:

> receiving a sensed an external ear canal pressure; and
> processing the sensed ear canal pressure to detect bruxism or temporomandibular joint disorders from the sensed ear canal pressure; and

generating an output signal indicating the detected bruxism or temporomandibular joint disorders

The implemented method may comprise detecting bruxism by:

identifying types of jaw movement from the sensed ear canal pressure;
determine if grinding or clenching jaw movements are present; and
detect bruxism based on a frequency of grinding or jaw clenching movements which exceeds a threshold.

The implemented method may comprise detecting temporomandibular joint disorders by:

identifying types of jaw movement from the sensed ear canal pressure;
determine if jaw closing movements are present;
determine a jaw movement range and jaw movement speed from pressure change amplitudes and rate of change of pressure changes; and
detect temporomandibular joint disorders based on a function of a jaw movement range and jaw movement speed.

These and other aspects of the disclosure will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a system for sensing bruxism or temporomandibular joint disorders;
Fig. 2 shows an example of pressure changes in the external ear canal during yawning;
Figs. 3a and 3b show an example of a method 200 for detecting TJD and bruxism;
Fig. 4 shows changes in pressure of the external ear canal during jaw movement during slow movement of the jaw and during fast jaw movement; and
Fig. 5 shows a duty cycle control of microphone and speaker modes.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for detecting bruxism or a temporomandibular joint disorder. A pressure sensor senses an external ear canal pressure and bruxism or a temporomandibular joint disorder are identified from the sensed ear canal pressure. An output signal indicates the detected bruxism or temporomandibular joint disorders so that remedial action can be taken.

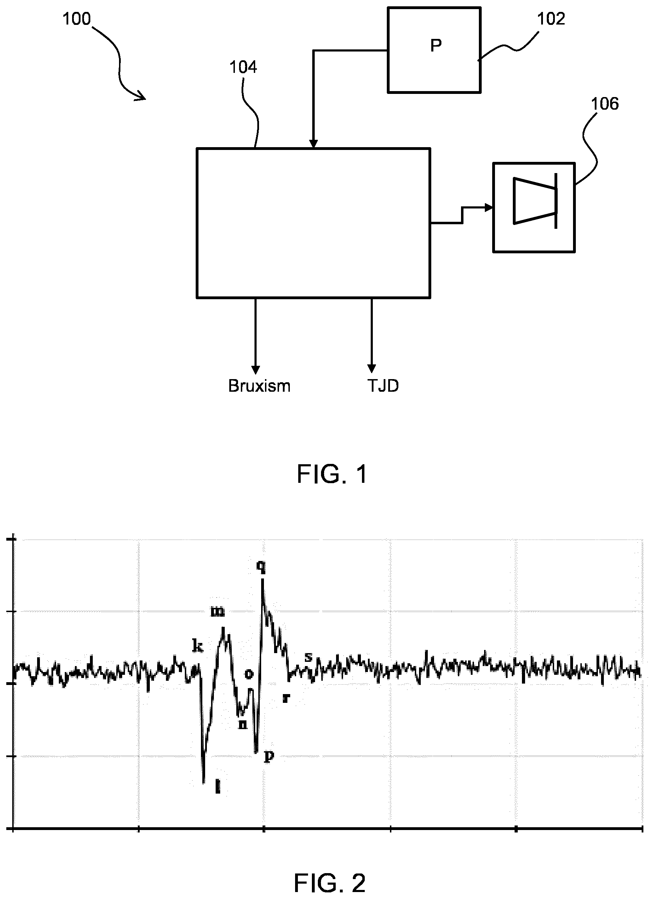

Fig. 1 shows the system 100, comprising a pressure sensor 102 for sensing an external ear canal pressure and a processor 104 for processing the sensed external ear canal pressure. Bruxism or temporomandibular joint disorders are detected from the sensed ear canal pressure, and an output signal ("Bruxism" or "TJD") is generated.

The pressure sensor 102 is for example in the form of an earplug. The external ear pressure is the pressure between the ear drum and an inserted earplug. In use, the earplug seals the ear canal from the external atmosphere. This essentially creates a closed chamber. The pressure sensor 102 is on one side of the chamber and the tympanic membrane is on the other side. The earplug may also be used for other purposes such as entertainment or communication purposes. The earplug is worn by the user during daily life and during sleep e.g., in the form of a so-called sleep band or snooze band.

There may be a single earplug or more likely there may be two earplugs inserted in both the ears. Fig. 1 shows a second earplug 106 represented as a speaker rather than a pressure sensor.

Thus, there may be a single pressure sensing earplug 102, or two pressure sensing earplugs, or one pressure sensing earplug and one loudspeaker earplug. In some embodiments as discussed below, the, or each, earplug may perform both pressure sensing and loudspeaker functions, and the same structure may be used for both functions (i.e. a speaker in output mode or in sensing mode). The system may however be implemented without any loudspeaker function, using pressure sensors.

A measure of oral pathologies is possible with a single earplug in a single ear, but the specificity of the measurement will be improved if both ears are measured. If both ears are monitored, the examination may be carried out with a single ear plug applied successively to the two ears or with two ear plugs applied simultaneously to both ears. Most preferably, two ear plugs are applied simultaneously.

However, all of these possible combinations of sensors may be considered generally to constitute a "pressure sensing arrangement" and are intended to fall within the general concept of the invention. The pressure sensing arrangement is for measuring air, i.e. atmospheric, pressure.

The pressure sensing arrangement is used to measure pressure changes in the external ear. Earplugs may be optionally integrated with an infra-red or inertial measurement unit (IMU) sensor to detect whether they are tightly inserted in the ear canal.

The processor 104 receives the pressure information from the external ear canal. The pressure information from the pressure sensing arrangement is then assessed to establish jaw movements.

A most simple implementation is a pressure sensor 102 in the form of an earplug or earbud (and both of these are intended to be considered as "earplugs") integrated with a pressure sensor. The pressure sensor may be a barometric MEMS pressure sensor or indeed any other pressure sensor. The earplug is used to continuously monitor pressure changes in the external ear.

Bruxism is characterized by frequent teeth grinding or clenching, which involves either side-to-side lateral movement (grinding) of the jawbones or tight closing (clenching) of the jawbones. In some cases, bruxism activity is rhythmic with bite force pulses of tenths of a second (similar to chewing), and in some cases it has longer bite force pulses of 1 to 30 seconds (clenching).

The grinding movement involves the TMJ of both the sides of the jaw; during grinding one half of the jaw (along with the corresponding TMJ) moves inwardly while the other half moves outwardly. Due to the close anatomical relationship between the jawbones and external ear canals, these activities produce changes in the dimensions of the external canal of both the ears, which results in characteristic changes in ear pressure.

Using experimental and simulation data it is possible to build a database of typical patterns of external ear canal pressure that are associated with teeth grinding or clenching due to bruxism.

Bruxism also involves frequent repetitions of these grinding or clenching actives. Therefore, monitoring the frequency of the teeth grinding or clenching episodes is also relevant. Based on clinical data, it is possible to derive a certain threshold (e.g. T1) of the duration of teeth grinding or clenching episodes to differentiate bruxism-related teeth grinding or clenching from other activities such as eating. Such a threshold could be also modified based on important demographic features such as age, sex etc. of the subject. Alternatively, the data can be used to train machine learning algorithms to classify bruxism.

Ear pressure changes can be also used to detect whether a person is awake or at sleep. Sleep is marked by a prolonged period of no or minimal activities in the oral cavity. Important oral activities such as phonation, swallowing (which both create known pressure signals in the earplugs) are conspicuously absent during sleep. Drowsiness before sleep is marked by frequent yawning. Yawning also produces characteristic changes in ear pressure. Therefore, yawing followed by a prolong period of no or minimal activities in the oral cavity can be used to differentiate awake and sleep states of an individual. This information can be then used to categorize bruxism episodes in sleep and awake bruxism.

Fig. 2 shows an example of pressure changes in the external ear canal during yawning (as pressure versus time).

It is known that, in many cases, bruxism can be reduced by using proper reminder techniques. Effectiveness of such techniques can be increased further if such reminders or notifications are provided to users in almost in real time during or immediately after a bruxism episode, and on a continuous basis.

Thus, once bruxism is detected based on analysis of the ear pressure as described above, an appropriate notification (the output "Bruxism") can be generated and delivered to the users. For awake bruxism, an earbud which includes a speaker (i.e. an earbud with pressure sensing capability and loudspeaker functionality) can be used to deliver appropriate audio notification to the user in real-time. The user's compliance to such reminders can be also monitored and can be used for user education. The user's response to such notifications can be used to adjust properties of the future notification to improve the effectiveness.

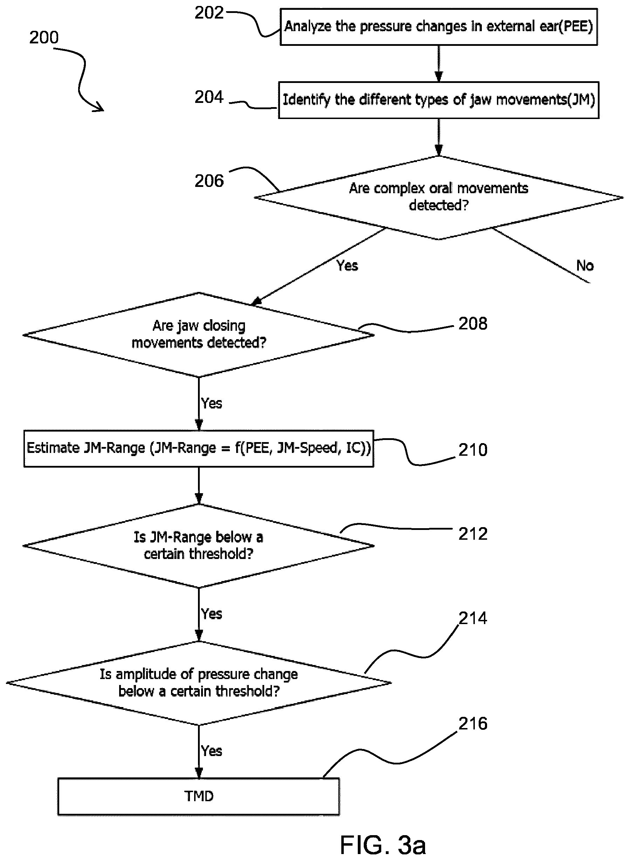

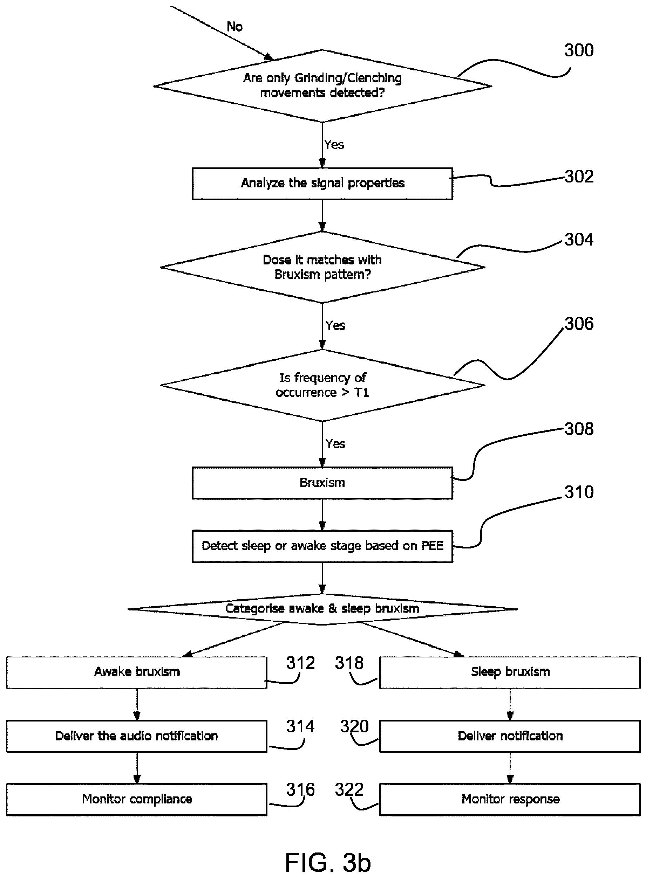

Figs. 3a and 3b show an example of a method 200 for detecting TMD and bruxism.

In step 202, pressure changes in the external ear are analyzed. These are termed "PEE". In step 204, different types of jaw movements "JM" are detected from that analysis of pressure changes.

In step 206, it is detected if complex oral movements are detected. The complex movements for example include swallowing and yawning.

The presence of complex movements may be indicative of TJD, whereas the absence of complex movements may be indicative of bruxism.

Basic jaw movements such as jaw up and down (clinching) and lateral movements of the jaw (grinding) may be considered to be simple movements.

More complex movements include yawning, eating and swallowing for example, and they include different combinations and sequences of these simple movements. These may be considered to be complex movements.

The pressure signal patterns may be used to classify and identify movements. For example Fig. 2 shows pressure changes in the external ear canal during yawning as explained above. The pattern includes a sequence of simple jaw movements. Bruxism instead will have only a simple movement in isolation which can be used for its identification.

The bruxism movement may result in a smoother, somewhat sinus-like increase and decrease in pressure, whereas TMJ has many more features.

Fig. 3a shows the steps when complex jaw movements have been detected.

In step 208, it is determined if these movements include jaw closing movements.

In step 210, for the jaw closing movements, a jaw movement range "JM-Range" is identified. JM-Range is the maximum distance between the two jaws.

To determine jaw movement, the amplitude of pressure changes may be measured. In general, the larger the jaw movement range, the higher the amplitude of the pressure change. Furthermore, the rate of change of pressure change may also be measured. For example, the rate of change in pressure can be used in combination with the total amplitude to establish the total jaw motion.

The relationship between the pressure changes in the external ear and the jaw movements (range and speed) can be expressed in a mathematical form as:

$$PEE = f(\text{JM-Range, JM-Speed, IC}) \qquad (\text{Eq. 1})$$

Where, PEE = Pressure in the external ear,

JM-Range = Jaw Movement range,
JM-Speed = Jaw Movement speed,
IC = Individual's characteristics (age, sex, height etc.,).

Based on empirical or experimental data, it is possible to derive a mathematical model to derive the range of jaw movement based on the measured PEE. Equation 1 in such case can be modified as:

$$\text{JM-Range} = f(PEE, \text{JM-Speed, IC}) \qquad (\text{Eq. 2})$$

Any changes in the amplitude, rate of change or smoothness of the jaw movement are used to assess the development of TJD.

In particular, in step 212 it is determined if the joint movement range is below a threshold. If so, it is also determined in step 214 if the amplitude of pressure change is below a threshold.

If both of these threshold comparisons are met, TJD is determined in step 216.

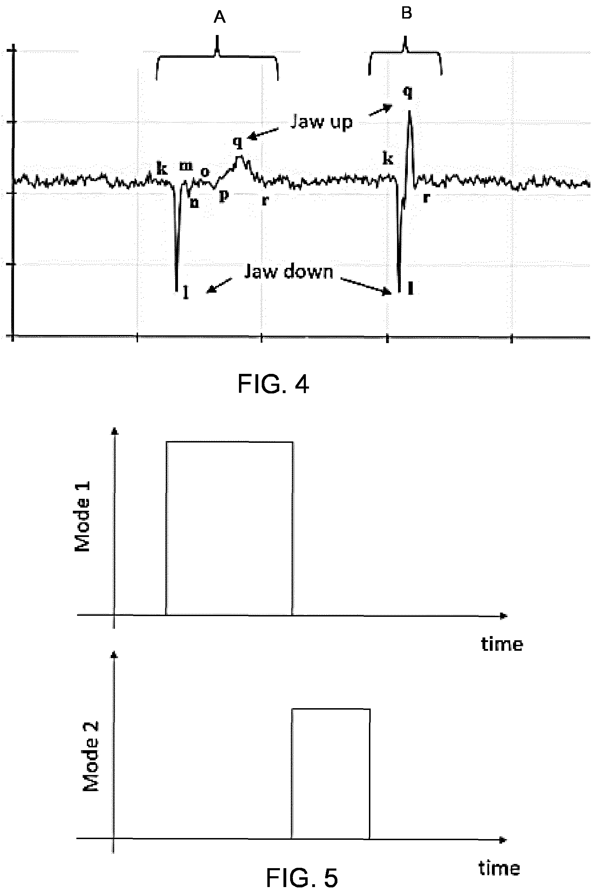

Fig. 4 shows changes in pressure of the external ear canal during jaw movement of the same individual in two different settings; segment A denotes slow movement of the jaw (a fast jaw drop and a slow jaw closure); segment B denotes fast jaw movement (a fast jaw drop and a fast jaw closure). It is evident that amplitude of pressure change depends on both range of jaw movement and speed of jaw of movement.

In general, a higher peak pressure will result from a larger and faster jaw movement.

In Fig. 4, the distance between points k and r in both examples denotes total time taken for one sequence of jaw up and down movement. This time can be used to calculate speed of jaw movement.

In the case of TJD, both the range and speed of jaw movement are restricted due to muscle guarding and associated pain. Therefore, pressure changes in case of TJD will have a slow rate of change of pressure (resulting in the more gentle slope of k-1 and p-q segments illustrated in segment A in Fig. 4) and reduced amplitude (of k-1 and p-q segment).

The muscles involved in the jaw drop are smaller, slower and much less powerful than those which are involved in pulling the jaw up. The speed of jaw movement has a less prominent change on pressure changes during the jaw drop compared to the jaw closure. The small notch in segment B in the 1-q segment denotes an abrupt change in pressure due to faster movement.

A large database of pressure changes in different conditions of TJD can be used to derive certain thresholds (e.g., if JM-Range is below a certain threshold) or train machine learning algorithms to detect TJD problems.

The TJD measurements may be made using an earplug integrated with pressure sensors and a microphone. The earplugs are used to monitor pressure changes in the external ear during jaw movements as described above and the microphone is additionally used to detect any click sound during jaw movements. The pressure and audio signals are then co-analyzed to find TJD.

The system may be enhanced with a pain sensor to detect pain during jaw movement, so that the detection of TJD covers all three classical features of TJD.

Fig. 3b shows the steps when complex oral movements have not been detected.

In step 300 (following step 206 of Fig. 3a), it is determined if only grinding or clenching movements are detected. If so, the signal properties are analyzed in step 302.

In step 304 it is determined if the signal properties match a bruxism pattern. If so, the frequency of these patterns is also determined in step 306 and compared with a threshold T1. If the frequency is greater than T1 bruxism is concluded in step 308.

The method then optionally also distinguishes between sleep bruxism and awake bruxism, by detecting the sleep or awake state in step 310 based on the pressure signals.

If awake bruxism is concluded in step 312, an audio or vibration/haptic notification is delivered in step 314 and it is monitored in step 316 if this notification has resulted in the bruxism ending.

If sleep bruxism is concluded in step 318, a notification is delivered in step 320 and it is monitored in step 322 if this notification has resulted in the bruxism ending.

This notification is for example a report to the user for the next morning.

The pressure sensing used in the system of the invention may be based on the re-use of acoustic transducers in the earplug. A loudspeaker may be reversed to function as a microphone. Thus a loudspeaker may be reconfigured as a microphone, thereby to act as an air pressure sensor. The displacement of the diaphragm can be used as air pressure sensor signal.

A single acoustic transducer may be operated in a microphone mode (Mode 1 - sound recording, ear pressure measurement) and in a speaker mode (Mode 2- sound generator). This may be implemented using duty cycle control as shown in Fig. 5. A controller is then used to switch the modes and control the sensitivity and duty cycle length/patterns of the two modes. The reconfigured speaker may be used to measure ear canal pressure and may also be used as a microphone to record tooth grinding or joint clicking sounds.

Alternatively, a system may have two acoustic transducers as are often already present in individual earphones, but reconfigured to function as one microphone and one speaker.

When a system has two earplugs, differential measurements may be made. Differential signal analysis may enable improved specificity of the diagnosis. Considering the pressure signals, constant differences between pressure signals in the left or right ear or differences which remain for several days can be interpreted as being related to ear/nose/throat/sinus pathologies. These fairly constant differences can be used to differentiate between the pathologies and the bruxism signals which are periodic in nature with frequencies of seconds or minutes. Furthermore any of these fairly constant differential signals can be used to differentiate between the ear/nose/throat/sinus pathologies and the TJD signals which will not show such a strong differential signal, if at all. Considering the audio signals, differentiation in either arrival time (phase) or amplitude of the sound signals may enable to better clarify (or even localize using arrival time shifts) the dental pathologies such as which teeth are grinding.

An oral healthcare routine, such as tooth brushing, may be used as a stimulus for measurement of TJD. In particular, jaw movement during tooth brushing, jetting, air flossing, insertion or removal of a brushing mouthpiece etc. may be used as a stimulus for measuring TJD.

Specific situations which induce a controlled jaw movement include:

opening of the mouth at the start of a tooth brushing session;

closing of the mouth at the end of a tooth brushing session;

opening of the mouth to insert a brushing mouthpiece and also to remove a brushing mouthpiece;

closing the mouth down onto a brushing mouthpiece after it has been inserted;

opening of the mouth to access the rear teeth for jetting or flossing;

possible side-to-side motion when jetting or flossing rear teeth.

Jaw movement during speech may also be used as a stimulus for measuring TJD. Speech could be when using the earplugs (with a microphone) as a communication device. The measured speech (intensity, spectrum) may for example be used to infer which movement is being done and this can be compared over time to check for any changes in the associated amount of jaw movement.

The system may be calibrated for example by requiring a patient to perform controlled jaw movements while taking the pressure (and optionally sound) measurements. For example, the patient may be asked to perform some simple manoeuvers such as swallowing, yawning and jaw drops. These actions create pressure changes in the external ear and which can be analyzed and used as a baseline for subsequent processing.

The system of the invention may be configured for detecting bruxism or TJDs or both. Thus, while the system is shown (in Figs. 3a and 3b) as enabling detection of both conditions, the invention may be applied to one of the two conditions only.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A system (100) for detecting and distinguishing between bruxism and temporomandibular joint disorders, comprising:

An air pressure sensor arrangement (102) configured to sense an external ear canal pressure; and
a processor (104) for processing the sensed ear canal pressure, wherein the processor is configured to:

detect bruxism or a temporomandibular joint disorder from the sensed ear canal pressure; and
generate an output signal indicating the detected bruxism or temporomandibular joint disorders,

wherein the air pressure sensor arrangement is configured for obtaining a differential pressure measurement from the two ears, either simultaneously or sequentially, and
wherein the processor is configured to use the differential pressure measurement to differentiate between (i) bruxism and temporomandibular joint disorders and (ii) ear/nose/sinus pathologies.

2. The system of claim 1, comprising a microphone for audio detection and the processor is for performing audio analysis.

3. The system of claim 2, wherein the processor is further configured to detect clicking or popping sounds or sounds emerging from teeth grinding or clicking sounds relating to temporomandibular joint disorders.

4. The system of any one of claims 1 to 3, wherein the air pressure sensor arrangement comprises a speaker which is operated in a microphone pressure sensing mode.

5. The system of any one of claims 1 to 4, wherein the air pressure sensor arrangement is part of a pressure sensing earbud or a pair of pressure sensing earbuds.

6. The system of any one of claims 1 to 5, wherein the processor is configured to analyze the sensed ear canal pressure to detect characteristic pressure patterns and repetition rates.

7. The system of any one of claims 1 to 6, wherein the processor is configured to detect bruxism by:

identifying types of jaw movement from the sensed ear canal pressure;
determine if grinding or clenching jaw movements are present; and

detect bruxism based on a frequency of grinding or jaw clenching movements which exceeds a threshold.

8. The system of any one of claims 1 to 7, wherein the processor is configured to detect temporomandibular joint disorders by:

identifying types of jaw movement from the sensed ear canal pressure;
determine if jaw closing movements are present;
determine a jaw movement range and jaw movement speed from pressure change amplitudes and rate of change of pressure changes; and
detect temporomandibular joint disorders based on a function of a jaw movement range and jaw movement speed.

9. The system of any one of claims 1 to 8, wherein the processor is configured to analyze the sensed ear canal pressure to detect whether the user is awake or asleep and is thereby able to distinguish between awake bruxism and sleep bruxism.

10. An oral care system comprising:

an oral care device; and
the system of any one of claims 1 to 9.

**Patentansprüche**

1. System (100) zum Erkennen und Unterscheiden von Bruxismus und Kiefergelenksstörungen, umfassend:

eine Luftdrucksensoranordnung (102), die dazu konfiguriert ist, einen äußeren Gehörgangdruck zu erfassen; und
einen Prozessor (104) zum Verarbeiten des erfassten Gehörgangdrucks, wobei der Prozessor konfiguriert ist zum:

Erkennen von Bruxismus oder einer Kiefergelenksstörung anhand des erfassten Gehörgangdrucks; und
Erzeugen eines Ausgangssignals, das den erkannten Bruxismus oder die Kiefergelenksstörungen anzeigt,

wobei die Luftdrucksensoranordnung so konfiguriert ist, dass sie eine Differenzdruckmessung von den beiden Ohren entweder gleichzeitig oder nacheinander erhält, und
wobei der Prozessor so konfiguriert ist, dass er die Differenzdruckmessung nutzt, um zwischen (i) Bruxismus und Kiefergelenksstörungen und (ii) Ohr/Nase/Sinus-Pathologien zu unterscheiden.

2. System nach Anspruch 1, das ein Mikrofon zur Audioerkennung umfasst, und wobei der Prozessor zum Durchführen von Audioanalyse dient.

3. System nach Anspruch 2, wobei der Prozessor weiter so konfiguriert ist, dass er Klick- oder Knallgeräusche oder Geräusche, die beim Zähneknirschen entstehen, oder Klickgeräusche im Zusammenhang mit Kiefergelenksstörungen erkennt.

4. System nach einem der Ansprüche 1 bis 3, wobei die Luftdrucksensoranordnung einen Lautsprecher umfasst, der im Mikrofon-Druckerfassungsmodus betrieben wird.

5. System nach einem der Ansprüche 1 bis 4, wobei die Luftdrucksensoranordnung Teil eines druckerfassenden Ohrhörers oder eines Paares druckerfassender Ohrhörer ist.

6. System nach einem der Ansprüche 1 bis 5, wobei der Prozessor so konfiguriert ist, dass er den erfassten Gehörgangdruck analysiert, um charakteristische Druckmuster und Wiederholungsraten zu erkennen.

7. System nach einem der Ansprüche 1 bis 6, wobei der Prozessor so konfiguriert ist, dass er Bruxismus erkennt durch:

Identifizieren von Kieferbewegungsarten anhand des erfassten Gehörgangdrucks;
Bestimmen, ob Knirsch- oder Presskieferbewegungen vorliegen; und
Erkennen von Bruxismus basierend auf einer Häufigkeit von Knirsch- oder Kieferpressbewegungen, die einen Schwellenwert überschreitet.

8. System nach einem der Ansprüche 1 bis 7, wobei der Prozessor so konfiguriert ist, dass er Kiefergelenksstörungen erkennt durch:

Identifizieren von Kieferbewegungsarten anhand des erfassten Gehörgangdrucks;
Bestimmen, ob Kieferschließbewegungen vorhanden sind;
Bestimmen eines Kieferbewegungsbereichs und einer Kieferbewegungsgeschwindigkeit aus Druckänderungsamplituden und einer Änderungsrate von Druckänderungen; und
Erkennen von Kiefergelenksstörungen basierend auf einer Funktion eines Kieferbewegungsbereichs und einer Kieferbewegungsgeschwindigkeit.

9. System nach einem der Ansprüche 1 bis 8, wobei der Prozessor so konfiguriert ist, dass er den erfassten Gehörgangdruck analysiert, um zu erkennen, ob der Benutzer wach oder schlafend ist, und dadurch in der Lage ist, zwischen Bruxismus im Wachzustand und Bruxismus im Schlaf zu unterscheiden.

10. Mundpflegesystem, umfassend:

eine Mundpflegevorrichtung; und
das System nach einem der Ansprüche 1 bis 9.

**Revendications**

1. Système (100) de détection et de distinction entre un bruxisme et des troubles articulaires temporo-mandibulaires, comprenant :

un dispositif capteur de pression d'air (102) configuré pour détecter une pression externe dans un conduit auditif ; et
un processeur (104) permettant de traiter la pression détectée dans un conduit auditif, dans lequel le processeur est configuré pour :

détecter un bruxisme ou un trouble articulaire temporo-mandibulaire à partir de la pression détectée dans un conduit auditif ; et
générer un signal de sortie indiquant le bruxisme ou les troubles articulaires temporo-mandibulaires détectés,

dans lequel le dispositif capteur de pression d'air est configuré pour obtenir une mesure de pression différentielle à partir des deux oreilles, simultanément ou successivement, et
dans lequel le processeur est configuré pour utiliser la mesure de pression différentielle de façon à distinguer (i) un bruxisme et des troubles articulaires temporo-mandibulaires et (ii) des pathologies au niveau des oreilles/du nez/des sinus.

2. Système selon la revendication 1, comprenant un microphone destiné à une détection audio et le processeur est conçu pour effectuer une analyse audio.

3. Système selon la revendication 2, dans lequel le processeur est en outre configuré pour détecter des sons de claquement ou de craquement, ou des sons produits par un grincement de dents, ou des sons de claquement liés à des troubles articulaires temporo-mandibulaires.

4. Système selon l'une quelconque des revendications 1 à 3, dans lequel le dispositif capteur de pression d'air comprend un haut-parleur qui est activé dans un mode de détection de pression de microphone.

5. Système selon l'une quelconque des revendications 1 à 4, dans lequel le dispositif capteur de pression d'air fait partie d'un écouteur à détection de pression ou d'une paire d'écouteurs à détection de pression.

6. Système selon l'une quelconque des revendications 1 à 5, dans lequel le processeur est configuré pour analyser la pression détectée dans un conduit auditif de façon à détecter des modèles de pression caractéristiques et des taux de répétition.

7. Système selon l'une quelconque des revendications 1 à 6, dans lequel le processeur est configuré pour détecter un bruxisme en :

   identifiant des types de mouvements de mâchoire à partir de la pression détectée dans un conduit auditif ;
   déterminant s'il existe des mouvements de grincement ou de crispation de la mâchoire ; et
   détectant un bruxisme sur la base d'une fréquence des mouvements de grincement ou de crispation de la mâchoire qui est supérieure à un seuil.

8. Système selon l'une quelconque des revendications 1 à 7, dans lequel le processeur est configuré pour détecter des troubles articulaires temporo-mandibulaires en :

   identifiant des types de mouvements de mâchoire à partir de la pression détectée dans un conduit auditif ;
   déterminant s'il existe des mouvements de fermeture de la mâchoire ;
   déterminant une plage des mouvements de mâchoire et une vitesse des mouvements de mâchoire à partir des amplitudes des variations de pression et du taux de variation des variations de pression ; et
   détectant des troubles articulaires temporo-mandibulaires sur la base d'une plage des mouvements de mâchoire et d'une vitesse des mouvements de mâchoire.

9. Système selon l'une quelconque des revendications 1 à 8, dans lequel le processeur est configuré pour analyser la pression détectée dans un conduit auditif de façon à détecter si l'utilisateur est éveillé ou endormi et peut par conséquent distinguer un bruxisme en phase d'éveil d'un bruxisme en phase de sommeil.

10. Système de soins bucco-dentaires comprenant :

   un dispositif de soins bucco-dentaires ; et

le système selon l'une quelconque des revendications 1 à 9.

FIG. 1

FIG. 2

200

202 — Analyze the pressure changes in external ear(PEE)

204 — Identify the different types of jaw movements(JM)

206 — Are complex oral movements detected?

Yes → No

Are jaw closing movements detected? — 208

Yes

Estimate JM-Range (JM-Range = f(PEE, JM-Speed, IC)) — 210

Is JM-Range below a certain threshold? — 212

Yes

Is amplitude of pressure change below a certain threshold? — 214

Yes — 216

TMD

FIG. 3a

No

Are only Grinding/Clenching
movements detected?    300

Yes

Analyze the signal properties    302

Dose it matches with
Bruxism pattern?    304

Yes

Is frequency of
occurrence > T1    306

Yes

Bruxism    308

Detect sleep or awake stage based on PEE    310

Categorise awake & sleep bruxism

| Awake bruxism 312 | 318 Sleep bruxism |
| Deliver the audio notification 314 | 320 Deliver notification |
| Monitor compliance 316 | 322 Monitor response |

FIG. 3b

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150039087 A1
- US 2019343452 A1
- EP 3936046 A1
- JP 3810026 B
- US 2020312321 A1